# EUROPEAN PATENT APPLICATION

(11) **EP 1 491 894 A2**
(43) Date of publication of application: **29.12.2004**
(21) Application number: 04014730.8
(22) Date of filing: 23.06.2004
(51) Int. Cl.: G01N 33/573, G01N 33/68, C12N 9/12, C12Q 1/42

(54) **Method for the determination of cell activation**

(30) Priority: 25.06.2003 US 606162
(71) Applicant: EPPENDORF AG, 22339 Hamburg (DE)
(72) Inventor: Remacle, José, 5020 Malonne (BE); Michel, Gaetan, 5170 Lesves (BE); Mainfroid, Veronique, 4300 Waremme (BE)
(74) Representative: Becker Kurig Straus

(57) **Abstract**

This invention provides a method for evaluation of the level of activation of cells through the quantification of the phosphorylated versus the non phosphorylated multiple proteins belonging to kinase cascades which is the result of the equilibrium between the kinase/phosphatase activities in the given cells. The invention also provides a method for quantification of the level of phosphorylation of enzymes and proteins involved into the pathways of cell activation or being the products of theses activations. In particular, the invention provides a method for the simultaneous quantification level of phosphorylation of multiple proteins of the same or different pathways present in the same cell extract.

## Description

### Field of the invention

This invention relates to a method for determining whether a cell is in an activated state or not, which method comprises assessing the equilibrium between kinase and phosphatase activities inside a cell. The present invention also pertains to a method for the quantification of the level of phosphorylation of enzymes and proteins involved in the pathways of cell activation or being the products of these activations. In particular, a method is provided for the simultaneous quantification of the phosphorylation level of multiple proteins of the same or different pathways present in the same cell.

### State of the art

One of the reactions of cellular systems to environmental changes, such as chemical, biological or physical stimuli, resides in activation of the cell, which is characterized by a complex interplay of signal transduction cascades involving post-translational modification of proteins. Among the variety of modifications, protein phosphorylation was found to be the most common mechanism for switching a protein from its inactive state to an active state, including phosphorylation on tyrosine, serine/threonine and/or histidine.

Phosphorylation/dephosphorylation are performed by kinase and phosphatase enzymes, which may exhibit an inter-correlated performance. Following a stimulating event, cell kinases and phosphatases are both transiently activated, leading to time-dependent fluctuations in the phosphorylation level of their cellular targets. These targets include the kinases and phosphatases themselves, or other enzymes, adaptor proteins, structural proteins and transcription factors.

Defective or inappropriate phosphorylation has been evidenced in many human disorders, which has been attributed in most cases to an imbalance of kinase/phosphatase activities. Therefore, monitoring both, kinase and phosphatase activities, are of high interest to obtain a better understanding of cellular activation mechanisms or its status.

The study of such complex cellular reactions is presently performed with technologies having, however, limited capabilities only, e.g. western blotting, kinase activity assays and ELISA methods.

Western blotting comprises separation of the proteins via electrophoresis and then transfer onto a membrane. Subsequently, proteins of interest are detected with antibodies specific for each of the proteins, in the case of phosphorylated proteins, phospho-specific antibodies. However, this process is quite time consuming and may be performed on one protein at a time only on each of the membranes.

In order to analyze proteins exhibiting similar molecular weights for post-translational modifications a procedure combining immunoprecipitation (IP) with western blotting is applied. In the case of phosphorylation on e.g. tyrosine or serine, anti-phosphotyrosine or anti-phosphoserine antibodies are utilized. Again, a disadvantage of this method resides in that each antigen of interest requires its own blot.

Kinase activity assays are another tool to study post-translational modifications of proteins, specifically phosphorylation events. These assays are generally performed by mixing a substrate with the enzyme containing solution in the presence of radio-labeled ATP for a certain period of time. The phosphorylated substrate is subsequently separated from the non-phosphorylated one and from other proteins via electrophoresis. The kinase activity is reflected by the amount of radioactivity incorporated into the substrate during a given incubation time.

ELISA assays are widely used particularly so as to follow receptor activation and/or to detect specific post-translational modifications of proteins, such as tyrosine phosphorylation. Examples include detection of the Heregulin-induced ErbB2 phosphorylation (Sadick et al. Analytical Chemistry 235 (1996), 207-214) and assay for VEGF-induced Flk-1/KDR phosphorylation. For ELISA assays, the cell lysate from each sample is added into wells of a 96-well plate pre-coated with an antibody against a desired antigen to allow binding of the desired protein onto the surface of plate. After the binding, the cell lysate is removed from the well and replaced with a specific antibody that is directly or indirectly conjugated with an enzyme. The amount of antigen of interest is then determined by the activity of the antibody-conjugate enzyme activity. Even though ELISA assays may be performed on 96-well plates and are designed to screen a large number of samples for a single antigen they are not well suited for an analysis of multiple antigens on a single 96-well plate.

In the recent past a new burgeoning field has emerged, functional proteomics. Here, the difference in the status of a cell is directly related to variations in the levels of proteins expressed. The methods being used for proteomic analyses combine 2D gel electrophoresis for separation with mass spectroscopy for identification of the respective proteins. However, these methods are quite time consuming and not optimized for high throughput analysis. They are also limited in that they are not suitable for use with transmembrane proteins, are technically difficult to perform, and have limited ability to detect low abundance proteins, such as those present in signal transduction pathways. A general and flexible technology, that can detect proteins and their interactions is currently not available in proteomics.

In the past, protein analysis has also been performed on protein arrays, which in general represent a tool for performing assays in high throughput. Protein arrays are patterned arrays of known biomolecules that can undergo a molecular recognition with specific proteins among a complex mixture of proteins in solution. So far, various concepts have been proposed for protein arrays to screen for antibodies, ligands and receptors that interact with the proteins of the arrays. Various protein chips are exemplified here under.

US-P-6,475,809 and 6,406,921 describe protein arrays and protein-coated substrates for the parallel, in vitro screening of biomolecular activity. These protein arrays are described to be particularly useful in high throughput drug screening and clinical diagnostics.

US-P-6,325,904 discloses microelectronic chips for the binding of protein-specific receptors without the use of any specific binding agents or molecules. The chip is described to be usable to detect, characterize and to quantify single molecules in solution such as individual proteins, complex protein mixtures, DNA or other molecules.

US-P-6,491,871 describes a synthetic strategy for the creation of large scale chemical diversity and the use of this solid-phase array for determining receptor-ligand binding affinity. Solid-phase chemistry, photolabile protecting groups, and photolithography are used to achieve light-directed spatially-addressable parallel chemical synthesis of a plurality of ligands. Ligand-arrays are used to screen fluorescently-labeled receptors.

US-A-20020177242 provides methods for adsorbing proteins to a substrate under a plurality of different selectivity conditions, and detecting the proteins retained on the substrate by desorption spectrometry. The methods are useful in biology and medicine, including clinical diagnostics and drug discovery.

WO-02084249 provides a method for determining the antibody specificity profile in an individual through the binding of the patient's antibodies comprising samples to the arrays that contain antigens and epitopes. This specificity profile reveals the individual's immune response to multiple antigens and/or epitopes of auto-antigens, allergens, graft antigens, etc..

US-A-2002168640 is directed to the formation of protein arrays through the use of nucleic acid/protein (NAP) conjugates, which allow the covalent attachment of proteins and the nucleic acids encoding them. By using vectors that include capture sequences that will hybridize to capture molecules on a nucleic acid array, the NAP conjugates including the proteins of interest are arrayed and used in a wide variety of applications.

Also, some protein arrays have been notified to be suitable for the detection of protein post-translational modifications.

WO-0214864 describes methods to elucidate signal transduction pathways and other biological processes governed by enzyme-mediated post-translational modifications. The array systems described are adapted for the investigation of the activity of enzymes involved in signal transduction pathways.

Further, WO-0157530 discloses a method to assess the status of a selected signal transduction pathway, i.e. the protein interactions and the phosphorylation events. This method includes applying a cell lysate to a series of binding reagents immobilized on a membrane in order to investigate the protein-protein interactions and the protein interaction network.

WO-0127624 provides methods for detecting protein modification and enzyme activity. The methods comprise contacting a sample containing a target protein with a plurality of capture molecules immobilized on a porous solid support and assessing the modification status of the immobilized target protein.

However, all of the methods of the prior art are not suitable for reliably and accurately giving a picture of the activation state of a cell.

### Object of the invention

Therefore, an object of the present invention is to provide a method for the evaluation, whether a cell is in an activated state.

Another object of the invention resides in providing a kit for determining, whether a sample cell is in a particular activated state or not.

### Summary of the invention

During the extensive studies leading to the present invention it has been found that an actual picture of the activation state of a cell may be obtained, when determining the equilibrium between kinase and phosphatase activities.

Therefore, the invention provides a method for evaluating the activation level of cells via the quantification of phosphorylated versus non-phosphorylated proteins belonging to the kinase cascades which is the result of the equilibrium between the kinase/phosphatase activities in a cell at a given time.

The invention also provides a method for the quantification of the level of phosphorylation of multiple enzymes and proteins involved in the pathways of cell activation or being the products of theses activations. In particular, the invention provides a method for the simultaneous quantification of the phosphorylation level of a variety of proteins of the same or different pathways present in the same cell extract.

None of the prior art documents discloses an evaluation of the level of cell activation in relation to specific pathways via the absolute quantitative determination of the phosphorylated levels of proteins participating in the cascade of activation.

### Brief description of the drawings and tables

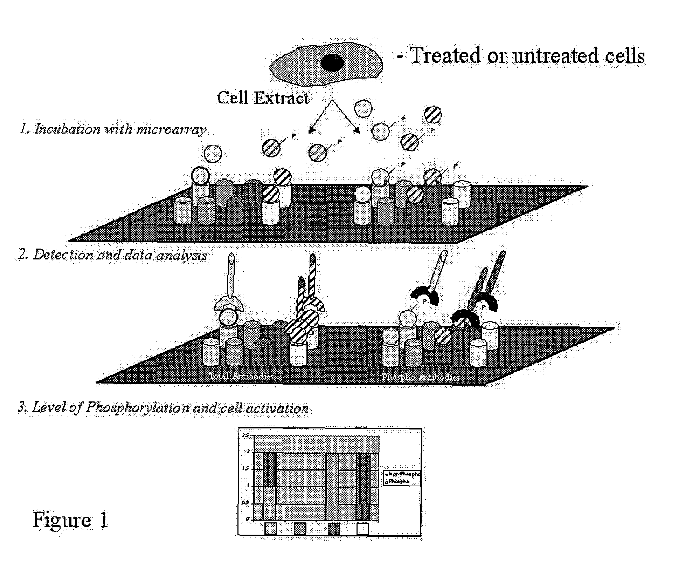

Fig. 1 illustrates the principle of the quantitative determination of the level of phosphorylated proteins, Step 1: Specific capture antibodies against phosphorylated and non phosphorylated proteins are immobilized on the surface of a solid support building an array; Step 2: target proteins from the cell lysate are captured on two separate arrays at specific positions; Step 3: A detection antibody specific of both the phosphorylated and non phosphorylated forms of the target protein is incubated on the left array, while a detection antibody specific of the phosphorylated form of the protein is incubated on the right array; Step 4: A secondary antibody labeled with cy3 is incubated on the arrays and a fluorescent signal is generated which indicates the phosphorylation level of the target protein;

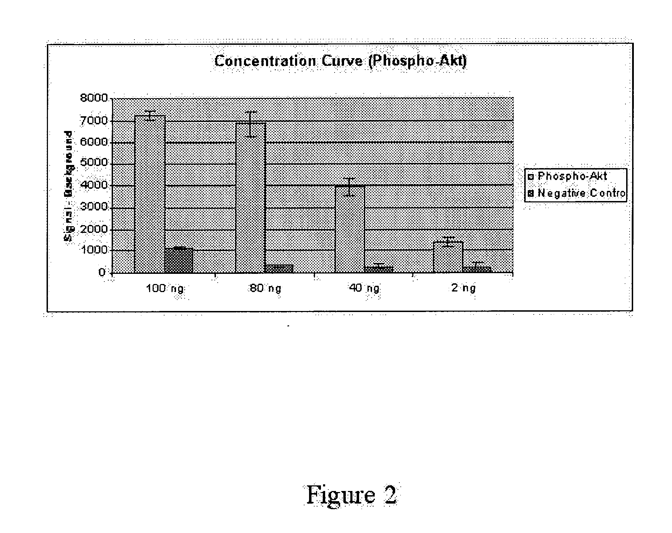

Fig. 2 illustrates the quantification of a concentration curve of phosphorylated Akt protein present in the solution (positive control) according to the example 1; The negative control is the assay performed in absence of phoshorylated Akt protein;
Fig. 3 illustrates the arrangement of an exemplary protein array for the MAP kinase pathway;
Fig. 4 illustrates the arrangement of an exemplary protein array for the PI3K/GSK-3 pathway;
Fig. 5 illustrates the arrangement of an exemplary protein array for the cell cycle pathway;
Table 1 lists proteins being phosphorylated and belonging to the serine/threonine kinase cascade;
Table 2 lists proteins being phosphorylated and belonging to the tyrosine kinase cascade;
Table 3 lists transcription factors being phosphorylated or not in response to the cell activation by kinase cascades.

### Detailed description of the invention

Unless otherwise defined, all technical and scientific terms used herein have the same meaning as is commonly understood by one person ordinary skilled in the art to which this invention belongs.

As used herein, "protein" encompasses polypeptides, oligopeptides and peptides.

As used herein, "protein modification" refers to addition of a peptidic or non-peptidic moiety to a protein that cannot be considered as the elongation of the peptidic chain of the protein. The addition of the peptidic or non-peptidic moiety can be performed in vivo or in vitro. The peptidic or non-peptidic moiety can be added to a pure protein or a protein or peptidic component of a complex containing such protein or peptide.

"Protein phosphorylation" refers to post-translational protein phosphorylation.

"Phosphorylation" includes phosphorylation on a protein residue being either a tyrosine, serine, threonine and/or histidine. Antibodies that can be used to detect these modifications can include phosphotyrosine-specific antibody, phosphoserine-specific antibody, and phospho-threonine-proline antibody, for example. Antibodies that may be used to detect these modifications also include antibodies specific to (a) phosphorylated residue(s) of a protein, or a fragment of the protein containing the phosphorylated residue(s).

"Biological sample" includes a biological fluid or a biological tissue or an extract thereof. Examples of biological fluids include urine, blood, plasma, serum, saliva, semen, stool, sputum, cerebral spinal fluid, tears, mucus, amniotic fluid or the like. Biological tissues are aggregates of cells, usually of a particular kind together with their intercellular substance that form one of the structural materials of a human, animal, plant, bacterial, fungal or viral structure, including connective, epithelium, muscle and nerve tissues. Examples of biological tissues also include organs, tumors, lymph nodes, arteries and individual cell(s).

"A control biological sample" is any biological sample taken as a reference compared to a "test biological sample". The control biological sample can be derived from a normal tissue and be compared to the test biological sample being a diseased tissue. Diseased tissue refers to a pathological condition in an organism resulting e.g. from infection or genetic defect, and is characterized by identifiable symptoms. The control and the test biological sample can be the same tissue but are derived from different organisms, or from the same tissue at different developmental or differentiation stages. The control biological sample can also correspond to untreated cells and the test biological sample to treated cells , wherein the treatment is physical, chemical, physiological or drug administration.

The term "drug" as used herein, should be interpreted broadly to include compounds of known efficacy and safety, drug candidates picked randomly from libraries of compounds, and drugs at every level of investigation therebetween.

Drugs include compounds which affects the level of activation of cells and could be modulators of kinase/phosphatase activities.

As used herein, "capture molecule" refers to a molecule, or complex or combination thereof, that is capable of specifically binding to one target protein, or to a family of proteins, or to one or more member (s) of a plurality of target proteins, or portion(s) thereof. The capture molecules are peptides, proteins, e.g., antibodies or part of it containing various parts of the heavy and/or the light chains of the antibodies, scaffold proteins or receptors or even nucleotides e.g. octamers being able to specifically recognize proteins. "Detection molecule" has similar properties as the capture molecule since it can recognize the presence of the target protein and lead to its detection through direct of indirect labeling.

"The capture molecule is capable of specifically binding to both a phosphorylated and a non phosphorylated form of a target protein" means that the capture molecule can bind to the target protein through specific interaction between the capture molecule and the peptidic portion of the target protein.

As used herein, "antibody" includes immunoglobulins which are composed of two light chains and two heavy chains linked by disulfide bounds and also fragments, such as Fab, (Fab)2, Fv or single variable region fragments (scFv).

As used herein, the term "receptor" refers to a molecule that has an affinity for a given ligand. Receptors may be naturally-occurring or synthetic molecules.

As used herein, the term "assessing" is intended to include quantitative and qualitative determination in the sense of obtaining an absolute value for the amount or concentration of the analyte present in the sample, and also of obtaining an index, ratio, percentage, visual and/or other value indicative of the level of analyte in the sample. Assessment may be direct or indirect and the chemical species actually detected need not of course be the analyte itself but may for example be a derivative thereof or some further substance.

"Array" means a support on which capture molecules are immobilized in order to be able to bind to the given specific protein or target. The array is preferentially composed of spots of capture molecules deposited at a given location on the surface or within the support or on the substrate covering the support. In one particular application of this invention, arrays of capture molecules are also provided on different supports as long as the different supports contain specific capture molecules and may be distinguished from each other in order to be able to quantify the specific target proteins. This can be achieved by using a mixture of beads having particular features and being able to be recognized from each other in order to quantify the bound molecules.

As used herein, the term "a group of structurally and/or functionally related proteins" refers to a group of proteins, at their natural status, that are structurally linked, located at the same cellular locations, e. g., cellular organelles, located in the same tissues or organs, expressed and/or be functional in the same biological stages, e. g., a particular cell cycle stage or developmental stage, or expressed and/or be functional in the same biological pathway, e. g., a particular metabolic pathway, signal transduction pathway, etc. or act as a regulator for a pathway activation or a biological function etc..

As used herein, "tissue" refers to a collection of similar cells and the intracellular substances surrounding them. There are four basic tissues in the body: 1) epithelium; 2) connective tissues, including blood, bone, and cartilage; 3) muscle tissue; and 4) nerve tissue.

As used herein, "organ" refers to any part of the body exercising a specific function, as of respiration, secretion or digestion.

As used herein, the terms "disease or disorder" refer to a pathological condition in an organism resulting from, e. g., infection or a genetic defect, and characterized by identifiable symptoms.

According to the present invention the activation state of a cell may be determined by quantitatively measuring the level of phosphorylated versus non-phosphorylated proteins belonging to the kinase cascades which is the result of the equilibrium between the kinase/phosphatase activities in a cell, and/or by quantifying the level of phosphorylation of multiple enzymes and proteins involved in the pathways of cell activation or being the products of theses activations. In particular, the invention provides a method for the simultaneous quantification of the phosphorylation level of a variety of proteins of the same or different pathways present in the same cell extract.

In general, the target proteins considered in the present invention are involved in one or several particular biological pathway(s), and/or belong to a group of proteins with identical or similar biological function(s), and/or are expressed at a particular stage of the cell cycle, expressed in a specific cell type, expressed in a tissue type, expressed in an organ type, or expressed in a developmental stage, proteins whose expression and/or activity is altered in a disease or disorder type or stage, proteins whose expression and/or activity is altered by drug or other treatments.

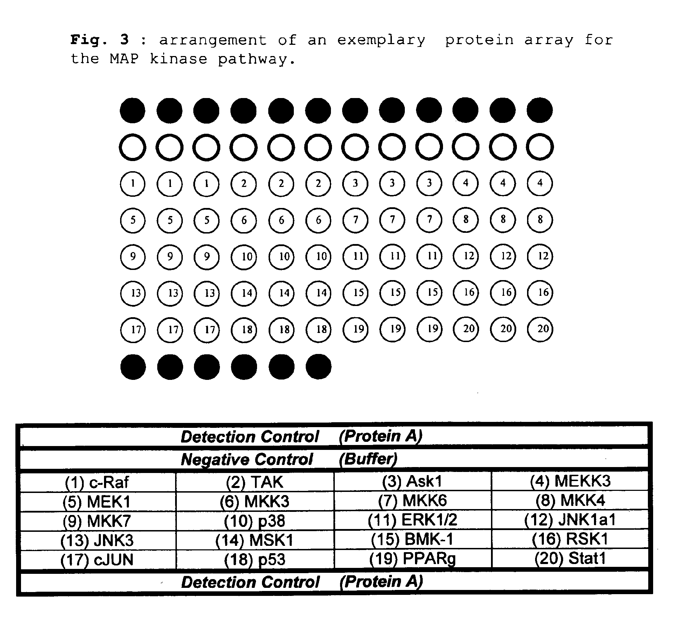

Also, the method of the present invention is used to quantitatively monitor the activity level of kinases and phosphatases of a given cascade resulting from an activation by an external stimulus. A list of such cascade proteins is presented in tables 1, 2 and 3. According to a preferred embodiment, the specific proteins to be detected are at least 3 proteins from the serine/threonine kinase cascade provided in table 1. According to an alternative preferred embodiment the proteins to be detected are at least 3 proteins from the tyrosine kinase cascade provided in table 2. According to another preferred embodiment the specific proteins to be detected is at least one protein, preferably at least 3 proteins, selected from table 3. According to another preferred embodiment the specific proteins to be detected is at least one protein, preferably at least 3 proteins, selected from figure 3

In another embodiment, the invention may also be used for the detection of a multitude of proteins from different kinase cascades and the resulting phosphoproteins. In general, protein phosphorylation occurs on an amino acid residue selected from the group consisting of tyrosine, serine, threonine and histidine.

It has been recognized that the mere quantification of one phosphorylated protein or the detection of several phosphorylated proteins by itself is not sufficient to determine, whether the cell is in an activated state or not and is surely not sufficient to estimate the level of the activation, since some proteins are phosphorylated even without activation or due to a general basal physiological activation, resulting in a given basic phosphorylation of some proteins in any case. Therefore, according to the present invention, either the activities of specific kinase/phosphatase enzymes are determined, which will give an overview of the cell's present activity to activate the cellular components, and/or the quantitative phosphorylation level of a variety of different cellular targets of the kinases/phosphatases is determined, resulting from the equilibrium between these kinases/phosphatases and providing a general overview about the present cell activation state. In consequence, by evaluating either of these information or by combining these data the actual activation of a cellular system may be determined.

According to a preferred embodiment, the method comprises quantitative determination of the level of phosphorylation at specific kinase and phosphatase residues acting on the same activation pathway or on the same target proteins of a specific activation pathway. The level of phosphorylation of the kinase/phosphatase is then converted into their level of activity and the ratio between the two kinase and phosphatase activities is an estimation of the cell activation level. Typical example of such invention is the estimation of the activities of the MAP kinase p44/42 (ERK1/2). ERK1/2 is a well known player in the antiapoptotic defense network of the cell. Activation of the Ras/MEK/ERK pathway was shown to phosphorylate Bad (Bcl-2 family member), allowing cells to protect from apoptosis. Thus ERK1/2 phosphorylation was demonstrated to stabilize key proteins in order to prevent degradation. However, the inactivation (or de-phosphorylation) of ERK1/2 by MAP kinase phosphatase (MKP-3) was shown to induce cytotoxicity (Rössig M., et al., JBC **275** (2000), 25502-25507).

Mitotic DNA checkpoints ensure that chromosomes are properly replicated and repaired before nuclear division. Mitosis is triggered by the cyclin-dependent kinase (CDK) Cdc2, which is inactivated by phosphorylation of tyrosine-15 due to kinases such as Weel and Mik1. Conversely, Cdc2 is activated by the protein phosphatase Cdc25 that dephosphorylates tyrosine-15, which is necessary for DNA damage checkpoint (Furnari B., et al, 1999, Mol Biol. Cell 10:833-845). This example clearly shows the importance of a tight regulation between kinases and phosphatases to ensure an ideal equilibrium in the cell process and activation and thus the necessity to quantify either both enzymes or the result of their opposite activities by determination of the level of phosphorylation of their target proteins.

According to a preferred embodiment the method comprises quantitatively determining the level of phosphorylated transcription factors optionally together with the level of phosphorylation of kinase(s)/phosphatase(s) involved in the signal transduction. Consequently, the present invention enables to give a quantitative evaluation not only of the level of the cascade activation but also of its physiologically relevant consequences on gene expression. A list of transcription factors activated through such kinase cascades and considered in the present invention as preferred representatives of transcription factors is given in table 3.

According to another preferred embodiment said specific cellular proteins is selected from the group consisting of: c-Raf, JNK1/2, ERK1/2, p38α, p90RSK, Hsp27, p70S6K, PKC, STAT1, STAT3, STAT5, Akt, GSK3β, mTOR, Bad, Creb, Src, AMPK, PAK1, IKB, p53, PLCγ, or from the group consisting of: Akt, BAD, GSK3β, eNOS, FKHR, AFX, PTEN, PDK1, mTOR, p70S6K, or from the group consisting of: FADD, Akt, ERK1/2, GSK3β, Bad, FKHR, JNK1/2, p53, IκB, BcI2, PKR, or from the group consisting of: p53, Rb, cdc2, Chk1, Chk2, cdc25, NBS1, GSK3β, p70S6K, or alternatively any combination of the representative of said groups.

The method according to the present invention is carried out on a non-membrane solid support bearing capture molecules for the specific proteins. In principle, any suitable "non-membrane" solid support may be used in the present invention, which may preferably consist of glasses, electronic devices, silicon supports, plastic supports, compact discs, metallic supports.

Any molecule, or complex or combination thereof, which is capable of specifically binding to a target protein, or one or more member(s) of a plurality of target proteins, may be used as a capture molecule. Preferably the capture molecules are macromolecules such as peptides, proteins, e.g., antibodies or receptors or a complex thereof. More preferably the capture molecules are antibodies or binding parts thereof, scaffold proteins or octamers capable to recognize specific proteins or parts thereof, or phospho-proteins. Even more preferably the capture molecules are antibodies, e.g., a polyclonal antibody, a monoclonal antibody, an antibody fragment retaining its desired binding specificity, or a combination thereof.

The amount of capture molecules immobilized on the support and required for detecting an amount of bound target proteins depends e.g. on the detection system used (in principle, the more sensitive the detection system, the less capture molecules required), on the binding affinity between the capture molecule and the target protein, on the expected relative amount of target proteins in the protein sample. The amount of capture molecules spotted on the support will be set by the skilled person so as to generate a detectable signal by the conventional means used in the laboratory. In a preferred embodiment, the amount of capture molecules will be in the range of about 10⁶ to about 10⁵ for getting spots of 0.4 mm in diameter. The size of the spots is preferably in the range of about 50 µm to about 500 µm in diameter, with the amount of capture molecules also be influenced by the size of the spots and the linear range of detection assay. Various means are used to spot the capture molecules. For preparing a protein micro-array, mechanical micro-spotting or ink jetting is the preferred preparation method (Schena M., et al, 1998, TIBTECH 16: 302-306).

When carrying out the present method, a biological sample is provided, which may be a purified or recombinant protein, or a fragment thereof, a mass of cells, a tissue, an organ, serum, blood, body fluids, or other sources, or lysates thereof, which potentially contain target proteins and for which it is desirable to test for the protein phosphorylation that has occurred in the sample. In principle, the cells of the sample are separated from each other by conventional means, such as e.g. trypsination etc.. If desired, the cells may be transferred into culture so as to subject them to particular conditions, such as exposing them to specific compounds, the activity on cells shall be determined. Alternatively, the cells essentially separated from each other may be directly used for further investigation. The cells are broken up and the cell extract/lysate may be subjected to a preliminary purification step to separate the proteinaceous material from other cellular components. Cell lysates may be prepared as described in Ausabel et al., eds., in the "Current Protocols in Molecular Biology" series of laboratory technique manuals. 1987-1994 Current Protocols, 1994-1998 John Wiley and Sons, Inc., which is incorporated herein by way of reference.

The sample is then contacted with the array. In particular, the capture molecules arrayed on the support are contacted with the biological sample containing proteins comprising target proteins or portions thereof that have possibly undergone the subject protein phosphorylation/dephosphorylation under conditions allowing binding of the target proteins to the respective capture molecules. The target proteins specific for a given population of capture molecules bind to the capture molecules and remain bound also after washing. Preferably, a target protein is bound to a capture molecule by an epitope or site that leaves any phosphate moiety on the target protein available for the subsequent detection step. Therefore, the non-phosphorylated and the phosphorylated forms will be bound likewise. To this end, the capture molecules are selected for the specificity and affinity of binding particular target proteins.

In a next step phosphorylated target proteins are detected by contacting the target proteins (bound to the capture molecules) with a detection molecule or a combination of detection molecules. In principle, the detection molecules may be conjugated with a detection means (e.g. enzymatic, fluorescent, colorimetric, chemi- or bio-luminescent, electrical detection means), or need to contact a label or tag, which is then detectable (indirect detection).

An example for a detection molecule is a phospho-specific antibody. Such phospho-specific antibody, e.g. phospho-tyrosine antibody, phospho-serine antibody, phospho-threonine antibody, or combinations thereof, may bear direct detection labels such as fluorescent or colorimetric labels, or may be linked to detection molecules that label indirectly through molecules such as biotin or other haptens such as fluorescein and digoxigenin etc. for amplification. Labeled streptavidin or labeled antibody against the hapten is then used for the detection. Also, a detection-ready antibody can be conjugated with enzymes such as alkaline phosphatase (AP), horse radish peroxidase (HRP) or others for direct detection.

In one embodiment, the specific antibodies react first on the antigen captured on the solid support and are subsequently detected through reaction with secondary labeled antibodies. Fluorescence molecules may be visualized without amplification of the signal. Alternatively, enzymes are added that provide amplification of the signal, and then either fluorescent molecules, chromogenic substrates, or chemiluminescent substrates are detected. In another embodiment the secondary antibody binds to a first molecule of a pair, being e. g. biotin, or haptens such as fluorescein, or digoxigenin, etc. This first molecule then reacts with the second molecule of the pair which provides any means for detection being either enzymatic, chemical, physical or electrical. Alternatively, further amplification is obtained by using enzymes that react upon substrates linked to another amplification molecule, e. g. biotin, fluorescein, or digoxigenin, etc., which in turn reacts with streptavidin or hapten antibody linked to enzymes that then react with fluorescent, chromogenic or chemiluminescent substrates. See Ausabel et al., eds., in the "Current Protocols in Molecular Biology" series of laboratory technique manuals. 1987-1994 Current Protocols, 1994-1998 John Wiley and Sons, Inc.

According to an alternative embodiment, the antibodies are coupled to nano-particles and detected as such preferably by their scatter absorption, diffusion properties, or by surface plasmon resonance or by their electrical properties, e.g. conductance or capacitance. In another preferred embodiment, metal nano-particles catalyze the precipitation of metal which is then detected.

The presence of the immobilized target protein may also be determined by other suitable physical or chemical means (See e. g., Yan et al., J Chromatogr. A., 808 (1-2) : 23-41 (1998)). In one application, the physical or chemical means comprise chemical or radio-isotopic label of the protein phosphate moiety. Alternatively, the physical or chemical means comprise any suitable analytical means, e. g., chromatographic, electrophoretic, protein sequencing, mass spectrometry and NMR means, for detecting the protein phosphate moiety.

In a preferred embodiment, the target protein captured and immobilized on a first array is put in contact with a detection molecule being capable of specifically binding to the phosphorylated target protein but not capable of specifically binding to the non-phosphorylated target protein, and the target protein immobilized on a second array is put in contact with a detection molecule being capable of specifically binding to the non-phosphorylated form of the target protein or even preferentially to both the phosphorylated and non-phosphorylated forms of the protein.

In another embodiment, the efficacy of detection of the phosphorylated versus the total or non-phosphorylated proteins is provided by reaction of the detection molecules with the same protein, phosphorylated or not, in a purified form and the experimental values obtained on a sample are corrected accordingly. In another embodiment, the ratios are compared with samples of cells being either not or sufficiently activated so that the experimental values of the ratio provide an evaluation of the cell activation compared to the two extreme cell situations.

According to another embodiment the same sample is incubated on two different arrays, the first being used for the quantification of the total proteins captured and the second for the quantification of the phosphorylated proteins, with the ratio between the two quantifications giving the level of activation of the cells. The two arrays may contain a plurality of identical capture molecules for the target proteins to be detected, and the level of target proteins is quantified on one array using detection molecules being specific for each of the proteins and the level of phosphorylation of target proteins is quantified on the other array using detection molecules being specific for the phospho-proteins.

Alternatively, the method according to the present invention comprises the steps of incubating a biological sample with two arrays present on the same support being composed of a plurality of capture molecules for the proteins to be detected, said capture molecules for said proteins being identical in both arrays, incubating the arrays with solutions containing two groups of detection molecules, the first one being contacted with the first array and being able of specifically binding to the phosphorylated target proteins but not to the non-phosphorylated target proteins, the second one being contacted with the second array and being able to specifically binding to the non-phosphorylated forms and possibly the phosphorylated forms of the target proteins, determining the level of phosphorylation of said immobilized target proteins, and evaluating the activation level of the cells from which the biological sample is derived.

Also, the method comprises the steps of incubating a biological sample with two arrays present on different supports containing a plurality of capture molecules for the proteins to be detected, said capture molecules for said proteins being identical in both arrays, incubating the arrays with solutions containing two groups of detection molecules, the first one being contacted with the first array and being able of specifically binding to the phosphorylated target proteins but not to the non-phosphorylated target proteins, the second one being contacted with the second array and being able to specifically binding to the non-phosphorylated forms and possibly phosphorylated forms of the target proteins, assessing the level of phosphorylation of said immobilized target proteins, and evaluating the activation level of the cells.

Specifically, the present method comprises the steps of a) contacting a sample containing or suspected of containing multiple target proteins with two distinct arrays, said arrays containing a plurality of capture molecules immobilized on a non-membrane solid support, said capture molecules being capable of specifically binding to both the phosphorylated and non phosphorylated forms of the target proteins, b) contacting the target proteins immobilized on the first array with detection molecules being capable of specifically binding to the phosphorylated target proteins but not capable of specifically binding to the non phosphorylated target proteins, and contacting the target proteins immobilized on the second array with detection molecules being capable of specifically binding to both the phosphorylated and non phosphorylated forms of the target proteins, and c) assessing the level of phosphorylation of said immobilized target proteins by measurement the signal ratio between the phosphorylated versus the total target proteins present in the same sample.

The phosphorylation level of any target protein or any plurality of target proteins present in cell is assessed by the present method by determining the signal ratio between the phosphorylated versus the total target proteins obtained on the two arrays according to the above methods, which gives a quantitative value of the level of phosphorylation for each target protein in the cells, among which kinases or phosphatases themselves. Their activity is associated with their level of phosphorylation and reflects the level of activation of the cells. The ratio may be corrected in order to take into consideration the variation in the efficiency of binding of the capture molecules to the phosphorylated or non-phosphorylated proteins. The determination of the correction ratio is best obtained by the double assay performed on purified phosphorylated proteins together or not with the non-phosphorylated proteins.

In a specific embodiment, a biological sample used as a control may be a normal tissue and a test biological sample may be a diseased tissue, wherein it is possible to evaluate the degree of activation of cells from both samples and to use this information for a subsequent assessment of a tissue exhibiting a diseased state or not. The diseased tissue may refer to a pathological condition in an organism resulting from infection or a genetic defect, and which may be pathologically or phenotypically characterized by identifiable symptoms. The control and the test biological samples may also be from the same tissue but from different organisms, or from the same tissue at different developmental or differentiation stages.

According to an alternative embodiment the control biological sample may be untreated cells and the test biological sample may be treated cells, which treated cells have been subjected to either of physical, chemical, physiological treatment, such as e.g. exposure to heat or drug administration. This enables the skilled person to look for compounds affecting the level of activation of the cells.

Where a comparison is made using a protein array, e.g. a comparison between a control array and a test array from a protein mixture of a particular condition or change in a condition, the control sample is derived from e. g. a normal tissue and the test sample from an altered tissue, e. g. a diseased tissue, or the control and test samples are from the same tissue but from different organisms, or from the same tissue at different developmental or differentiation stages. The organism in a comparison is e.g. wild type, diseased, knockout or transgenic. The array also represents different tissues or cells from the same body. E.g., the control sample may be untreated cells and the test sample may be treated cells. The treatment may comprise various biological, physical or chemical actions, such as exposing the sample to a drug treatment, whether a known approved drug or a test drug. The treatment also comprises such treatments, e.g. as administration of a growth factor, UV irradiation, or other drugs, chemicals or therapies to treat a disease or condition or to cause a change in a condition.

In an embodiment, the present method is used to determine the protein phosphorylation profile of a particular tissue such as epithelium tissue, connective tissues, including blood, bone, and cartilage, muscle tissue and nerve tissue, or a particular organ, i. e. any part of the body exercising a specific function, such as respiration, secretion or digestion.

In yet another embodiment, the present method is used to determine the protein phosphorylation profile of a particular disease or disorder, such as infection, neoplasm (neoplasia), cancer, an immune system disease or disorder, a metabolism disease or disorder, a muscle or bone disease or disorder, a nervous system disease or disorder, a signal disease or disorder, or a transporter disease or disorder.

According to another embodiment the level of activation of cells will be directly related to the presence of an externally added compound, which may be a drug. The efficacy of the compound for acting on the cell activation will be evaluated accordingly. The compound efficacy will be compared to the chemical structure of the compound in order to detect potential trends into the chemical parameters influencing the cell activation. In another embodiment, compounds activity will be evaluated for inhibition of cell activation in the presence of an external stimulus.

According to another embodiment, the level of activation of cells will be directly related to the presence of a compound being added to the cells or the cells extract considered as sample and the influence of said compound on the level of phosphorylation of kinases/phosphatases or their targets involved in specific activation pathways will be evaluated through determining the level of phosphorylation of at least one protein of the pathway.

The present invention also pertains to a kit for the evaluation of the activation level of cells by quantification of the level of phosphorylation of multiple specific cellular proteins participating in signal transduction in response to a stimulus. The kit comprises at least one support containing a plurality of immobilized capture molecules, said capture molecules being able to specifically bind to both the phosphorylated and non-phosphorylated forms of each cellular protein; two solutions, each containing groups of detection molecules, the first one being able of specifically binding to the phosphorylated target proteins but not to the non phosphorylated target proteins, the second one being able to specifically bind to the non-phosphorylated forms and possibly to the phosphorylated forms of target proteins; means for assessing the level of phosphorylation of said immobilized target proteins. According to a preferred embodiment either or both the capture molecules and the detection molecules are antibodies.

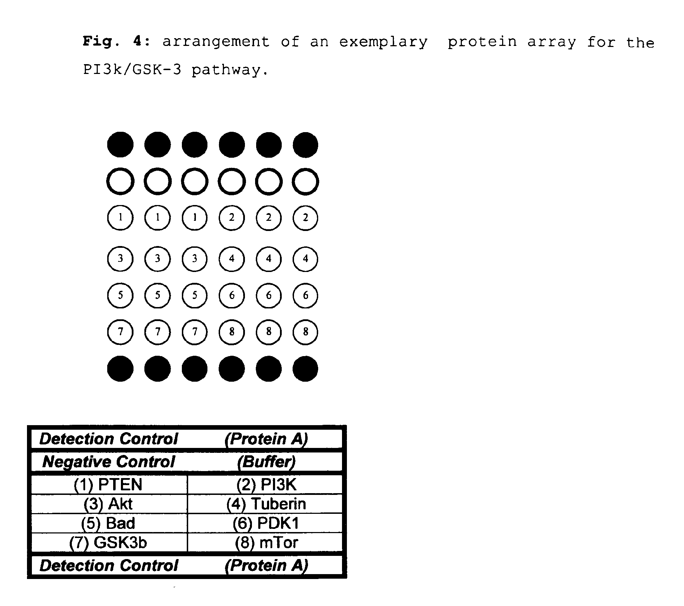

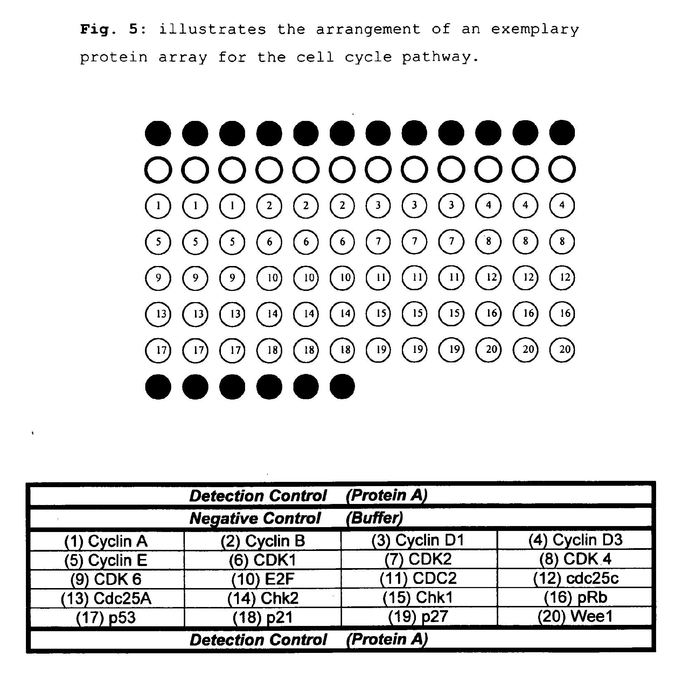

The support provided in the kit contains a plurality of capture molecules capable to specifically bind at least 5 of the proteins indicated in table 1 and/or at least 5 of the proteins from table 2 and/or at least 5 proteins indicated in table 3. A preferred kit contains a support with capture molecules specifically binding proteins as exemplified in figures 3, 4 and 5.

In still another aspect, the present invention is directed to a kit for an absolute quantification of the level of phosphorylation of multiple targets within a sample, comprising the steps of a) contacting a sample containing or suspected of containing multiple target proteins with two distinct arrays, said arrays comprising a plurality of capture molecules immobilized on a non-membrane solid support, said capture molecules being capable of specifically binding to both the phosphorylated and non-phosphorylated forms of the target proteins, and b) contacting the target proteins immobilized on the first array with detection molecules being capable of specifically binding to the phosphorylated target proteins but not to the non-phosphorylated target proteins, and contacting the target proteins immobilized on the second array with detection molecules being capable of specifically binding to both the phosphorylated and non phosphorylated forms of the target proteins, and c) assessing the level of phosphorylation of said immobilized target proteins by measurement the signal ratio between the phosphorylated versus the total target proteins present in the sample. The level of phosphorylation gives a quantification value for the activation level of the cells from which the sample originates.

The present kit is preferably used in a high throughput format. In one embodiment, the kit comprises a plurality of arrays of multiple capture molecules immobilized on the same or different solid supports, each of said arrays being identical or having capture molecules able of specifically binding to the same proteins or to the same group of structurally and/or functionally related target proteins.

Consequently, the present method involves the absolute quantification of the level of phosphorylation in multiple targets within a sample.

The following examples are given for illustrative purposes and should not be considered to limit the invention.

### Example 1

### Quantification of a concentration curve of phosphorylated Akt protein.

Hybridization chambers were placed onto diaglass functionalized slides (EAT, Namur, Belgium) spotted with antibodies specific of the protein to be detected, in this case anti-Akt antibodies. The spotting was performed with a split pin or a pin and ring or a plain pin. Each spot contained between 0.5 and 10 nl depending on the type of pin used and its diameter.

The diameters of the spots were between about 250 and 450 µm. For each antibody, 4 spots were present on the array, and positive and negative controls of spotting and a negative hybridization control for testing the non specific binding of the protein on non specific antibodies were included.

Similar slides were contacted separately with 50 µl of different biological samples, consisting of 100, 80, 40, or 2 ng of purified phosphorylated Akt protein. Incubation was performed for 2h in a thermomixer (Eppendorf, Germany) at 1400 rpm, 22°C.

The slides were washed 4 x 1 min in Washing Buffer (EAT, Namur, Belgium). The slides were then incubated for 1 h with 500 µl of a specific rabbit detection antibody against phospho-Akt diluted 500 x in Blocking Buffer (EAT, Namur, Belgium). The slides were washed 4 x 1 min in Washing Buffer. The slides were then incubated for 45 min at room temperature with a conjugated antibody (anti-rabbit Cy3) diluted 1000 x in Blocking Buffer. The slides were washed 4 x 1 min in Washing Buffer and then rinsed twice in water. The slides were scanned in a laser confocal scanner "ScanArray" (Packard, USA) at a resolution of 10 µm.

The results show that the assay is quantitative since the signals correlate with the amount of Akt protein present in the sample. The assay is also specific due to the absence of any signal in the absence of phosphorylated Akt protein (negative control). The assay is very sensitive with a detection limit of phospho-Akt of 2 ng/50 µl which corresponds to 4 x 10⁻¹⁴ mol/50 µl or 8 x 10⁻¹⁰ M.

### Example 2

### Quantification of the level of phosphorylated versus total Akt

The assay was performed as described in example 1, but two identical arrays were present on each slide. The capture antibodies spotted on the left and right arrays were the same, but the detection antibodies were either specific to the phospho-Akt (right arrays) or to the total Akt (left arrays). Four different mixes were prepared as follows: (1) 40 ng of purified Akt and 40 ng of purified phospho Akt; (2) 40 ng of purified Akt and 20 ng of purified phospho Akt; (3) 40 ng of purified Akt and 10 ng of purified phospho Akt; (4) 40 ng of purified Akt and 5 ng of purified phospho Akt. In this experiment, four slides were used. Both arrays (left and right) on each slide were incubated with one of the mixes described above (ex: slide 1 with mix 1, etc.). The detection step was done as follows: the left arrays on each slide were incubated with a biotinylated anti-total-Akt antibody diluted 100x. The right arrays were incubated with a biotinylated anti-phospho-Akt antibody diluted 100x. An anti-biotin conjugated Cy3 antibody was incubated with each array for the detection (dilution on the left array : 1000x; dilution on the right array : 100x). The intensity of the spots on each array was then quantified and the results clearly show decreasing signal intensities correlating with decreasing concentrations of phospho-Akt on the right arrays. The calculated ratios of phospho vs. total Akt directly relates to the known concentration used in the four different mixes.

### Example 3

### Quantification of total Akt protein in HepG2 cells under normoxia and hypoxia.

The objective of this experiment was to detect the quantity of total Akt in HepG2 cells incubated in both normoxia and hypoxia for 5 or 16 hours. For this experiment, the slides were processed as described in example 1.

The quantity of proteins present in each cell lysate was measured by the Bradford technique. This value was used to normalize the signals obtained after quantification. 10 µl of cell lysates were diluted in 50 µl Blocking Buffer. Four conditions were tested: normoxia 5h, hypoxia 5h, normoxia 16h, hypoxia 16h. The slides were incubated for 2h at 22°C, on a thermomixer (1400 rpm). Following washing, the slides were incubated with an anti-Akt antibody. An anti-rabbit Cy3 is used for the revelation.

The results showed no variation in total Akt level in both normoxia and hypoxia after 5h. However, there is an 37% increase of total Akt after incubation of the cells in normoxia for 16 h, while a 24% decrease of total Akt following incubation of cells in hypoxia for 16 h was observed. These results correlate very clearly with results obtained in Western Blotting and ELISA assays, done in parallel.

### Example 4

### Specific quantification of multiple phosphorylated or non phosphorylated proteins on arrays

For this experiment, slides were spotted with the following antibodies: anti-Erk1/2, anti-Akt, anti-HSP27, anti-p38, anti-Jun, anti-ATF-2 and anti-p53. Each antibody is present in 4 spots.

Each slide contained 2 identical arrays. The two arrays were incubated with the same samples possibly containing the proteins to be detected. The left array was then incubated with a mixture of 7 detection antibodies directed against the 7 proteins to be detected. The right array was incubated with a mixture of 7 detection antibodies directed against the phospho-specific form of the 7 proteins to be detected. The assay was performed as in examples 1 and 2. An anti-biotin-Cy3 antibody was used for the revelation on both arrays. The samples consisted of each of the 7 proteins to be detected, in their non-phosphorylated or phosphorylated form, or combinations thereof.

In first assays, where each protein was incubated individually on the arrays. These multiplex experiments allows to control the specificity of the 7 capture antibodies, as only the capture antibody specific for the purified protein present in the sample generated a signal. It also shows the specificity of the phospho-specific detection antibodies for each of the 7 phosphoproteins. Examples of correction coefficients for the detection of the phospho versus the total proteins were of 3.8 6.5 for Akt and Erk, respectively.
Since the detection was found specific for each of the protein, the array was then used for the determination of the phosphorylation state of these 7 proteins.

### Example 5

### Specific quantification of multiple proteins of a kinase cascade present in a biological sample on an array

This experiment was performed on slides containing a double array as presented in example 4. In this multiplex experiment, the phosphorylation status of the following proteins was assessed : Erk1/2, Akt, HSP27, p38 and jun. Jurkat cells were treated with PMA (phorbol 12-myristate 13-acetate) at varying concentrations (0.1 to 1000 ng/ml) for 10 minutes. Cells were then lysed and cell lysates were put in contact with the arrays. As in example 2, total and phosphorylated proteins were quantitatively determined on the left and right arrays, respectively. The results obtained show that the amount of Erk1/2 remains constant, while the levels of phosphorylation at threonine 185 and tyrosine 187 increase with the amount of PMA used for the cell stimulation.

### Example 6

### Kinetic of cell activation through phosphorylation quantification

The experiment was performed on slides containing a double array as presented in example 4. Jurkat cells were treated with 8 different concentrations of anisomycin. Eight cell lysates were prepared and put into contact with the multiplex arrays. The protein chips allowed to simultaneously quantify the level of phosphorylation of p38 and JNK1/2 due to the effect of anisomycin on cells.

The results showed that JNK1/2 and p38 phosphorylation in Jurkat cells are dependent on the levels of stimulation. The levels of total JNK1/2 and p38 remain constant while the levels of the corresponding phospho forms increase with the anisomycin concentration. For JNK1/2, the ratios of Phospho vs. total forms are of 0.33 and 0.88 respectively for anisomycin concentrations of 0.01 and 1 µM, whereas for p38 the ratios are of 0.33 and 1.0 respectively for anisomycin concentrations of 1 and 100 µM.

### Example 7

### Evaluation of an activation state of Hela cells following stimulation with PMA

Hela cells were grown to 80% confluence in DMEM/high glucose medium supplemented with 10% FBS and antibiotics. Medium is replaced with serum-free medium and phorbol 12-myristate 13-acetate (PMA) is added for one hour at a final concentration of 100nM. Cell extracts from non stimulated and PMA-stimulated cells are prepared with the lysis buffer from Biosource (Belgium) according to the manufacturer recommendations.

A series of diaglass functionalized slides (EAT, Namur, Belgium) were spotted as described in example 1 with pan antibodies (recognizing both phosphorylated and non phosphorylated forms of their corresponding proteins) specific for the following proteins: c-Raf, JNK1/2, ERK1/2, p38α, p90RSK, Hsp27, p70S6K, PKC, STAT1, STAT3, STAT5, Akt, GSK3β, mTOR, Bad, Creb, Src, AMPK, PAK1, IKB, p53, PLCγ. Slides are then processed as in example 1.

Two slides with two identical arrays each were contacted, respectively, with protein extracts from non-stimulated Hela and PMA-stimulated Hela cells. In each case, 70µg total proteins are diluted to a final volume of 50µl with binding buffer (EAT, Belgium). The slides are then processed as in example 1, and detection is performed on one array of each slide using a mix of biotinylated pan antibodies, each being specific for one of the above-mentioned proteins, and on the other array using a mix of biotinylated phospho-specific antibodies, each recognizing the phosphorylated form of one of the above-mentioned proteins. These mixes contain one antibody specific for each of the above-mentioned proteins. An anti-biotin-Cy3 antibody is then added as in previous examples.

The spots intensities were quantified and the comparison of the signals obtained on the two arrays from each slide allowed to get information about the activation state of the test samples. Moreover, comparing the information obtained separately for Hela and PMA-treated Hela allowed to get information about the influence of PMA treatment on different activation cascades in Hela cells.

### Example 8

### Analysis of the PI3K/Akt pathway in HepG2 cells under normoxia and hypoxia

The same experiment as in example 3 was conducted, but the arrays differed in their antibodies composition, and the detection uses different antibodies.

For this experiment, a series of identical arrays are spotted with pan antibodies specific for the following proteins: Akt, BAD, GSK3β, eNOS, FKHR, AFX, PTEN, PDK1, mTOR, p70S6K. Samples from each of the four conditions described in example 3, namely normoxia 5h, hypoxia 5h, normoxia 16h and hypoxia 16h, are contacted with the two arrays from a same slide. Left arrays from each slide are then contacted with a mix of biotinylated pan antibodies, each being specific for one of the above-mentioned proteins, and right arrays with a mix of biotinylated phospho-specific antibodies, each recognizing the phosphorylated form of one of the above-mentioned proteins. These mixes contain one antibody specific for each of the above-mentioned proteins. An anti-biotin-Cy3 antibody is then added as in previous examples.

The spots intensities are quantified and the comparison of the signals obtained on the two arrays from each slide allows to get information about the activation state of each of the tested samples. The comparison of data obtained on the 4 different slides allowed to get information about the effect of hypoxia on the PI3K/Akt cascades in HepG2 cells.

### Example 9

### Analysis of the apoptosis cascade in Hela Cells treated with nocodazole

For this experiment, a series of identical arrays are spotted with pan antibodies specific for the following proteins: FADD, Akt, ERK1/2, GSK3β, Bad, FKHR, JNK1/2, p53, IκB, Bcl2, PKR.

Extracts from Hela cells +/- treated with 3µM nocodazole for 5 or 20 hours are prepared as in example 7. Aliquots of cell extracts from each test condition are contacted with two arrays from a same slide. Left arrays from each slide are then contacted with a mix of biotinylated pan antibodies, each being specific for one of the above-mentioned proteins, and right arrays with a mix of biotinylated phospho-specific antibodies, each recognizing the phosphorylated form of one of the above-mentioned proteins. These mixes contain one antibody specific for each of the above-mentioned proteins. An anti-biotin-Cy3 antibody is then added as in previous examples.

The spots intensities are quantified and the comparison of the signals obtained on the two arrays from each slide allows to get information about the activation state of each of the tested samples. The comparison of data obtained for non treated and nocodazole treated Hela cells showed the effect of the drug on the apoptosis cascade. in these cells.

### Example 10

### Analysis of the cell cycle control in Hela cells treated with hydroxyurea

For this experiment, a series of identical arrays are spotted with pan antibodies specific for the following proteins: p53, Rb, cdc2, Chk1, Chk2, cdc25, NBS1, GSK3β, p70S6K.

Extracts from Hela cells +/- treated with 5mM hydroxyurea for 16 hours are prepared as in example 7. Aliquots of cell extracts from each test condition are contacted with two arrays from a same slide. Left arrays from each slide are then contacted with a mix of biotinylated pan antibodies, each being specific for one of the above-mentioned proteins, and right arrays with a mix of biotinylated phospho-specific antibodies, each recognizing the phosphorylated form of one of the above-mentioned proteins. These mixes contain one antibody specific for each of the above-mentioned proteins. An anti-biotin-Cy3 antibody is then added as in previous examples.

The spots intensities are quantified and the comparison of the signals obtained on the two arrays from each slide allows to get information about the activation state of each of the test samples. The comparison of data obtained for non treated and hydroxyurea treated Hela cells showed the effect of the molecule on the cell cycle control in these cells.

### Example 11

### Individual analysis of phospho-proteins in Hela cells

For this experiment, eight identical arrays are spotted on two slides following the spotting procedure described in previous examples. Each array is composed of triplicate spots of capture antibodies specific for the following proteins: Erk1/2, Akt, Hsp27 and p38, as well as positive and negative detection controls. Slides are then processes as in previous examples. Eight-well hybridization chambers (Grace Bio-Labs, Inc.) are then applied onto each slide to enable subsequent processing of each array individually.

Extracts from Hela cells +/- treated with PMA are produced as in example 7, and aliquots from each cell extract are contacted with four of the eight arrays on each slide. The four top arrays correspond to non-stimulated Hela, while the four bottom arrays correspond to PMA-stimulated Hela.

One slide is then contacted with biotinylated pan antibodies, each being specific for one of the above-mentioned proteins, while the other slide is contacted with biotinylated phospho-specific antibodies, each recognizing the phosphorylated form of one of the above-mentioned proteins. In this example, the antibodies are not added as a mix, but individually to two of the eight arrays, one top and one bottom array. An anti-biotin-Cy3 antibody is then added as in previous examples.

The spots intensities are quantified and the comparison of the signals obtained on the corresponding top and bottom arrays allows to get information about the effect of PMA on the phosphorylated and non-phosphorylated forms of each protein individually in Hela cells. The comparison of the corresponding arrays from the two slides allowed to get information about the phosphorylation of each of the four studied proteins.

### Example 12

### Analysis of the phosphorylated residues in multiple proteins

The same experiment as in example 5 is conducted, but the detection step of the phosphorylated protein forms uses different antibodies. Seven series of duplicate slides are contacted with aliquots from the same samples: one of the duplicate slides in each series is contacted with extracts from non-stimulated Jurkat cells, while the other slide is contacted with extracts from PMA-stimulated Jurkat cells. The left arrays from all 14 slides are contacted with a mix of five antibodies, respectively specific for the total forms of Erk1/2, Akt, HSP27, p38 and jun. The right arrays from each slide are contacted with phospho-specific antibodies that differ for every duplicate: the right arrays from each of the seven duplicate series are contacted with antibodies against phospho-tyrosine residues, phospho-serine residues, phospho-threonine residues, or combinations thereof. All combinations are assayed, leading to a total of seven different phospho-antibody pools.

All these total and phospho-specific antibodies are biotinylated, and detection is performed using an anti-biotin-Cy3 antibody as in previous examples.

The spots intensities are quantified and the comparison of the signals obtained on the two arrays from each slide allows to get information about the activation state of each of the tested samples. The comparison of data obtained on the 2 slides in each duplicate series allows to get information about the effect of PMA on the phosphorylation of the five studied proteins in Jurkat cells. The comparison of the seven series of duplicate slides allowed to get information about the different residues that are phosphorylated upon PMA stimulation in Jurkat cells.

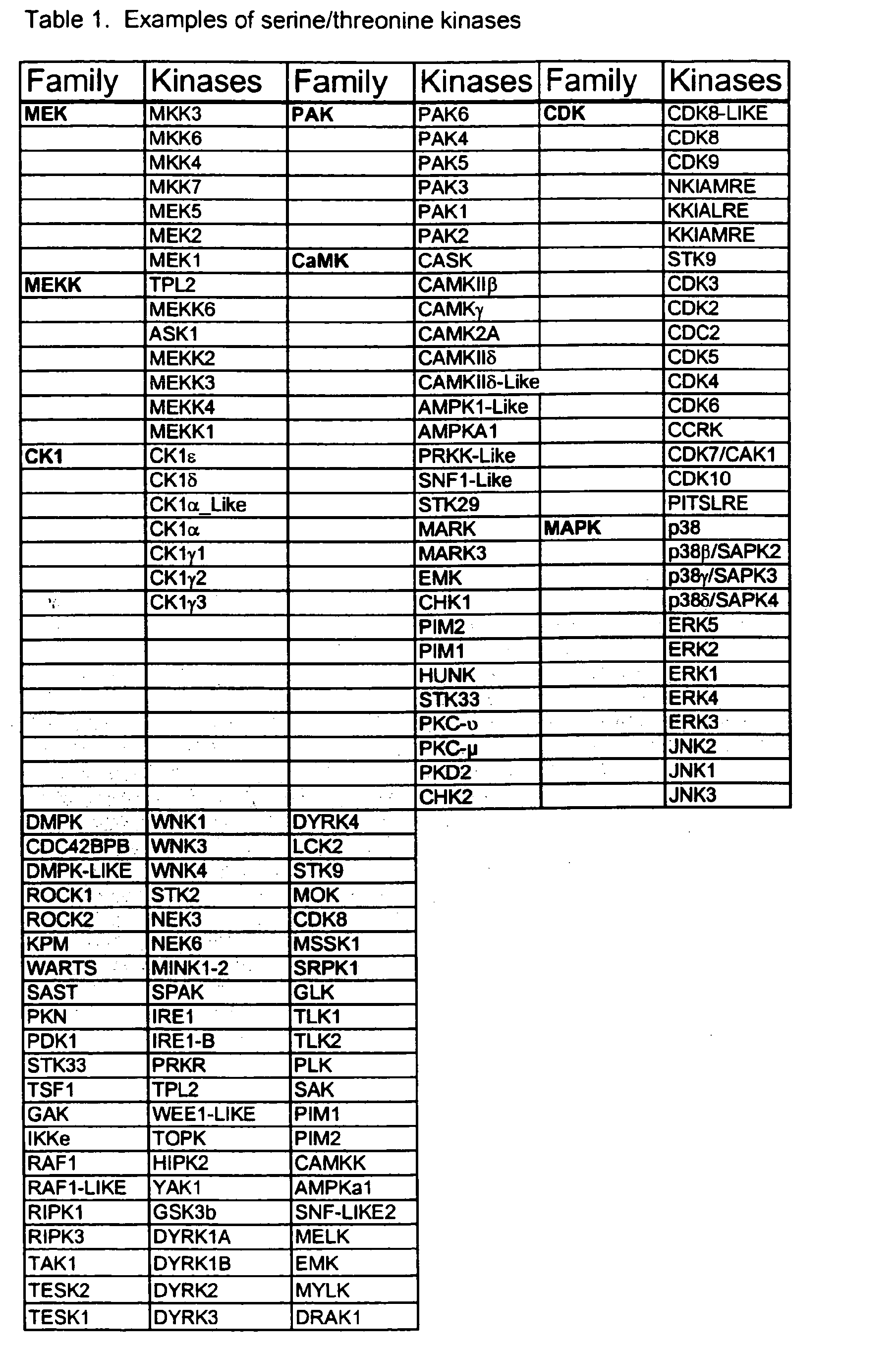

**Table 2.**

| Examples of tyrosine kinases | | | | |
|---|---|---|---|---|
| A. Cytoplasmic tyrosine kinases | | | | |
| Family | Kinases | Family | Kinases | |
| **JAK** | JAK1 | **ABL** | ARG | |
| | JAK2 | | ABL | |
| | JAK3 | **TEC** | ITK | |
| | TYK2 | | TEC | |
| **SYK** | ZAP70 | | BTK | |
| | SYK | | TXK | |
| **SRC** | LYN | | BMX | |
| | HCK | **FES** | FER | |
| | BLK | | FES | |
| | LCK | **ACK** | TNK1 | |
| | FYN | | ACK1 | |
| | FGR | **FAK** | FAK | |
| | SRC | | PYK2 | |
| | YES1 | | | |
| | MATK | | | |
| | CSK | | | |
| | FRK | | | |

| B. Receptor tyrosine kinases | | | | |
|---|---|---|---|---|
| DDR1 | RET | ERBB4 | EPHA3 | LMR2 |
| DDR2 | TIE | ERBB2 | EPHA5 | LMR1 |
| ROS | TEK | ERBB3 | EPHA4 | |
| AXL | FLT3 | EGFR | EPHA7 | |
| MER | FGFR4 | | EPHA8 | |
| SKY | FGFR3 | | EPHB1 | |
| MET | FGFR2 | | EPHB2 | |
| RON | FGFR1 | | EPHB3 | |
| RYK | VEGFR1 | | EPHB4 | |
| TRKA | VEGFR2 | | EPHB6 | |
| TRKB | VEGFR3 | | EPHA1 | |
| TRKC | KIT | | EPHA2 | |
| MUSK | CSF1R | | INSR | |
| CCK4 | PDGFRA/B | | IGF1R | |
| ALK | | | INSRR | |
| LTK | | | | |
| ROR1/2 | | | | |

**Table 3.**

| Examples of transcription factors activated by phosphorylation/dephosphorylation | |
|---|---|
| Kinases | Transcription factors |
| ERK 1/2 | Elk-1, Stat 1/3, Ets-1, ER, c-Myc, SRF, CREB |
| SAPK/JNK | c-Jun, ATF-2, Elk-1, p53, DPC4 |
| p38 MAPK | ATF-2, MEF2C, Elk-1, Myc/Max, Stat1, CREB, CHOP |
| ERK5/BMK | MEF2C |
| p90^{rsk} | c-Fos, SRF, CREB |
| MSK1 | CREB |
| JAKs | STATs |
| PKA | CREB, SAF-1, GATA-4, SOX9, HNF-4, AR |
| PKB/Akt | forkhead, AFX |
| GSK3β | AP1, β-catenin, C/EBPα, CREB, HSF-1, Myc, NFAT, NFκB |
| ALKs | SMADs |
| CaMK | Ets-1, CREB, STAT1 |

## Claims

1. A method for the evaluation of the activation state of cells, which comprises the step of determining the equilibrium between the activities of kinase/phosphatase enzymes on proteins participating in signal transduction into cells or quantifying determining the level of phosphorylation of cellular proteins participating in signal transduction into cells.

2. The method according to claim 1, wherein the proteins participating in signal transduction belong to a cascade of phosphorylation leading to the activation of at least one transcription factor.

3. The method according to any of the claims 1 to 3, wherein the proteins participating in signal transduction comprise at least one transcription factor.

4. The method according to claim 1, wherein the proteins are selected from at least one of: c-Raf, JNK1/2, ERK1/2, p38α, p90RSK, Hsp27, p70S6K, PKC, STAT1, STAT3, STAT5, Akt, GSKβ, mTOR, Bad, Creb, Src, AMPK, PAK1, IκB, p53, PLCγ, eNOS, FKHR, AFX, PTEN, PDK1, FADD, Bcl2 and PKR, Rb, cdc2, Chk1, Chk2, cdc25, NBS1, GSK3β, p70S6K.

5. The method according to claim 1, wherein the level of phosphorylation of the proteins is detected on a non-membrane solid support bearing capture molecules for the specific proteins by comparing the total amount of the fixed proteins to the phosphorylated amount of said specific proteins.

6. The method according to claim 5, wherein the non-membrane solid support is selected from the group consisting of glasses, electronic devices, silicon supports, plastic supports, compact discs, metallic supports.

7. The method according to claim 1, wherein the same sample is incubated on two arrays,
(i) wherein the first one is for the quantification of the total proteins fixed and the second for the quantification of the phosphorylated proteins, the ratio between the two quantifications giving an evaluation of the activation of the cells; and/or
(ii) wherein said first and second arrays comprise a plurality of identical capture molecules for said specific cellular proteins, said method further comprising contacting said specific cellular proteins on said first array with one detection molecule for one of said specific cellular proteins and contacting said specific cellular proteins on said second array with one detection molecule specific for one of said phosphorylated specific cellular proteins; and/or
(iii) wherein said first and second arrays comprise a plurality of identical capture molecules for said specific cellular proteins, said method further comprising contacting said specific cellular proteins on a series of said first arrays, and contacting each first array respectively with one of the detection molecules specific for said specific cellular proteins, and contacting said specific cellular proteins on a series of said second arrays, and contacting each second array respectively with one of the detection molecules specific for said phosphorylated specific cellular proteins; and/or
wherein said first arrays and said second arrays are present on the same non-membrane solid support; and/or
wherein said first arrays and said second arrays are present on different non-membrane solid supports, and/or
wherein the two arrays contain a plurality of identical capture molecules for the target proteins to be detected, and the level of target proteins is quantified on one array using detection molecules being specific for each of the proteins and the level of phosphorylation is quantified on the other array using detection molecules being specific for the phospho-proteins.

8. The method according to any of the preceding claims comprising the steps of :
- incubating a biological sample with two arrays present on the same or different support being composed of a plurality of capture molecules for the proteins to be detected, said capture molecules for said proteins being identical in both arrays,
- incubating the arrays with solutions containing two groups of detection molecules, the first one being contacted with the first array and being able of specifically binding to the phosphorylated target proteins but not to the non-phosphorylated target proteins, the second one being contacted with the second array and being able to specifically bind to non phosphorylated forms and possibly phosphorylated forms of the target proteins,
- determining the level of phosphorylation of said immobilized target proteins, and
- evaluating the activation level of the cells.

9. The method according to claims 8, wherein the data obtained on the phosphorylated proteins are corrected by a coefficient provided by analysis of a purified phosphoprotein.

10. The method according to any of the claims 8 or 9, wherein the capture molecules and/or the detection molecules are antibodies or binding parts thereof, scaffold proteins or octamers being able to recognize specific proteins or part of it, or phospho-proteins.

11. The method according any of the preceding claims, wherein the specific proteins to be detected are
(i) at least 3 proteins from the serine/threonine kinase cascade provided in table; and/or
(ii) at least 3 proteins from the tyrosine kinase cascade provided in table 2; and/or
(ii) at least 3 proteins provided in table 3; and/or
(iii) wherein at least one of the proteins to be detected is a protein as indicated in table 3; and/or
(iv) wherein at least one of the proteins to be detected is a protein as indicated in figure 5.

12. The method according to claim 1, wherein the phosphorylation occurs on an amino acid residue selected from the group consisting of tyrosine, serine, threonine and histidine.

13. The method according to claim 1, wherein the proteins are either involved in a biological pathway, belong to a group of proteins with identical or similar biological function, are expressed in a stage of cell cycle, expressed in a cell type, expressed in a tissue type, expressed in an organ type, or expressed in a developmental stage, proteins whose expression and/or activity is altered in a disease or disorder type or stage, or proteins whose expression and/or activity is altered by drug or other treatments.

14. A method for the evaluation of the activation level of cells, wherein the level of phosphorylation of proteins in a test biological sample as compared to a control biological sample is quantified, which comprises:
i) assessing the quantification of phosphorylated proteins of a test biological sample through the method of claim 2;
ii) assessing the quantification of phosphorylated proteins of a control biological sample through the method of claim 2; and
iii) comparing the protein phosphorylation status obtained in step i) with the protein phosphorylation status obtained in step ii) to identify the level of activation of proteins in said test biological sample.

15. The method of claim 14, wherein the control biological sample is a normal tissue and the test biological sample is a diseased tissue.

16. The method of claim 15, wherein diseased tissue refers to a pathological condition in an organism resulting from infection or genetic defect, and is **characterized by** identifiable symptoms.

17. The method of any of claims 14 to 16, wherein the control and the test biological samples
(i) are from the same tissue but from different organisms, or from the same tissue at different developmental or differentiation stages; or
(ii) the control sample is untreated cells and the test biological sample is treated cells.

18. The method of claim 17 wherein the treatment is physical, chemical, physiological or drug administration.

19. The method of claim 14, wherein the control and the test samples are extracts from the same cells but said test sample is the extract treated in the presence of a compound to be tested.

20. The method according to the preceding claims for screening of molecules affecting the level of activation of the cells.

21. The method according to claim 20, wherein the activity of the molecules is quantified by their effect on the level of activation of the cells.

22. A kit for the evaluation of the activation level of cells by quantification of the level of phosphorylation of multiple specific cellular proteins participating in signal transduction in response to a stimulus, which kit comprises:
- at least one support containing a plurality of immobilized capture molecules, said capture molecules being able to specifically bind to both the phosphorylated and non phosphorylated forms of each cellular protein, and
- two solutions, each containing groups of detection molecules, the first one being able of specifically binding to the phosphorylated target proteins but not to the non phosphorylated target proteins, the second one being able to specifically bind to the non phosphorylated forms and possibly to the phosphorylated forms of target proteins;
- means for assessing the level of phosphorylation of said immobilized target proteins and
evaluation of the activation level of the cells.

23. The kit according to claim 23, wherein
(i) the capture molecules are antibodies; and/or.
(ii) wherein the detection molecules are antibodies.

24. A support containing a plurality of capture molecules, said capture molecules being able to
(i) specifically bind at least 5 of the proteins from Table 1; and/or
(ii) specifically bind at least 5 of the proteins from Table 2; and/or
(iii) specifically bind at least 5 of the proteins from Table 3; and/or
(iv) specifically bind at least 5 of the proteins from figure 5.
